# EUROPEAN PATENT SPECIFICATION

(11) **EP 1 213 972 B1**
(45) Date of publication and mention of the grant of the patent: **02.05.2003**
(21) Application number: 00958855.9
(22) Date of filing: 08.09.2000
(51) Int. Cl.: A23L 1/03, A23L 1/307, A61K 38/16, A61K 38/00

(54) **COMPOSITION COMPRISING AN ALPHA-AMYLASE INHIBITOR AND AT LEAST ONE PHYSIOLOGICALLY ACCEPTABLE COMPOUND CAPABLE OF REDUCING INTESTINAL ABSORPTION OF "FAST SUGARS"**
ZUSAMMENSETZUNG DIE EINEN ALPHA-AMYLASE INHIBITOR UND MINDESTENS EINE PHYSIOLOGISCH AKZEPTABELE KOMPONENTE, DER DIE INTESTINALE ABSORPTION VON "FAST SUGARS" REDUZIEREN KANN, ENTHÄLT
COMPOSITION COMPRENANT UN INHIBITEUR D'ALPHA-AMYLASE ET AU MOINS UN COMPOSE PHYSIOLOGIQUEMENT ACCEPTABLE, CAPABLE DE REDUIRE L'ABSORPTION INTESTINALE DE SUCRES RAPIDES

(30) Priority: 08.09.1999 GB 9921229
(43) Date of publication of application: 19.06.2002
(73) Proprietor: Med-Eq AS, Sentrum, 3101 Tonsberg (NO)
(72) Inventor: MORTENSEN, Nils Christian, c/o Med-Eq AS, Sentrum, 3101 Tonsberg (NO)
(74) Representative: Campbell, Neil Boyd
(86) International application number: GB0003472
(87) International publication number: WO01017369

(56) References cited:
- EP-A- 0 372 522
- WO-A-00/43036
- US-A- 4 010 258
- US-A- 5 612 039
- SATHE S K ET AL: "Effects of germination on proteins, raffinose oligosaccharides, and antinutritional factors in the Great Northern beans (Phaseolus vulgaris L.)." JOURNAL OF FOOD SCIENCE 1983 DEP. OF NUTR. & FOOD SCI., UNIV. OF ARIZONA, TUCSON, ARIZONA 85721, USA, vol. 48, no. 6, pages 1796-1800, XP002153102
- MARTIN-CABREJAS M A ET AL: "Modifications to physicochemical and nutritional properties of hard-to-cook beans (Phaseolus vulgaris L.) by extrusion cooking." JOURNAL OF AGRICULTURAL AND FOOD CHEMISTRY 47 (3) 1174-1182 1999 DPTO. QUIMICA AGRICOLA, GEOLOGIA Y GEOQUIMICA, CVII, FAC. DE CIENCIAS, UNIV. AUTONOMA DE MADRID, 28049 MADRID, SPAIN. TEL. 34-91-3973831. FAX 34-91-3973830. E-MAIL MARIA.MARTIN(A)UAM.ES, XP002153103
- S. NAGY ET AL.: "Tropical and Subtropical Fruits. Composition Properties and Uses." 1980 , AVI PUB. , WESTPORT, US. XP002153197 page 172 -page 174

## Description

The present invention relates to compositions capable of reducing or preventing the uptake of excess sugars by the body. Such compositions find particular use as a dietary supplement, e.g. for maintaining a particular weight condition, for losing weight or for treating conditions such as obesity.

Overweight is an ever increasing problem. Although there are a number of factors which contribute to this, the main cause is an excess of sugar in the diet (any sugar which doesn't get burned up is stored as fat in the body). Since a high sugar intake very often leads to reliance on sweet-tasting foods, this exacerbates the problem.

Three types of nutrients are indispensable in the diet: glucides, proteins and lipids. A balanced diet involves the right intake level of each nutrient. The glucides, which comprise "fast" sugars (sugar, sweets, cakes, biscuits, pastries, chocolate, etc.) and "slow" sugars (pasta, bread, cereal, rice, potatoes, dried vegetables, etc.) generally represent about 55% of the calorie intake compared to 30% of lipids, and only 12% of proteins. In cases where the level of sugar intake exceeds normal levels, the hormone insulin (produced by the pancreas to reduce sugar level in the blood) transforms the sugar (both "fast" and "slow") into fat which leads to weight gain. Lack of physical exercise or activity adds to the problems associated with a high sugar diet.

In addition to weight gain, high levels of sugar in the diet have been linked to some types of cancer. For example, it has been suggested that those eating large quantities of sugar, pasta or white bread are at increased risk of polyps, colon and stomach cancer.

Insulin has also been the focus of much research. Excessive production of insulin by the pancreas can result in an increase in triglycerides and thickening of the blood vessels and the walls of blood veins which may lead to cardiovascular disease. Over-production of insulin can also result in "burning out" of the pancreas which is then unable to produce insulin. This can lead to diabetes.

Once food has been ingested, it goes through a series of biological processes before it is assimilated by the body. During digestion, food is converted into assimilable form principally through the action of various enzymes in the alimentary canal. A small amount of digestion takes place in the mouth through the action of the enzyme salivary alpha-amylase. However, starches and other carbohydrates are not exposed to this enzyme for any great period of time before they reach the stomach. Hydrochloric acid present in the stomach effectively inhibits starch digestion until the food exits the stomach and enters the small intestine.
Almost all complex carbohydrate digestion takes place in the duodenum. Here, pancreatic alpha-amylase provides for the break-up of starches ("slow" sugars) into monosaccharides ("fast" sugars) which are able to cross the intestinal barrier into the blood stream. In the small intestine, fats are absorbed.

Methods currently used for dietary control include a well-balanced diet with reduced calorie intake and/or physical activity or exercise. However, low-carbohydrate diets are restrictive in terms of choice of foods, are hard to manage and inevitably difficult to adhere to. Physical activity, unless carried out regularly, has little effect on weight control.

Thus there exists a need to provide an alternative means of dietary control.

We have now developed a dietary formulation comprising natural ingredients which work together to capture excess sugars and to reduce or prevent their storage in the body as fat. This formulation overcomes the problems of low-carbohydrate diets since it changes how the body absorbs carbohydrate calories without the need for any change to the normal diet.

In the Journal of Food Science, Volume 48 (1983), Sathe et al. describes Great Northern Bean (Phaseolus Vulgaris) germination. The bean, from which Phaseolamin™ is extracted, additionally comprises the sugars sucrose, stachyose and raffinose.

Viewed from one aspect the invention provides an orally administrable composition comprising at least one alpha-amylase inhibitor together with at least one physiologically acceptable compound capable of reducing intestinal absorption of "fast" sugars, compound selected from the group consisting of inulin derived from chicory, fructooligosaccharides and mixtures thereof. Optionally, the composition according to the invention may further comprise a fat inhibitor.

Preferred compositions according to the invention include those comprising:
(a) from 0.01 to 40% by weight of an alpha-amylase inhibitor;
(b) from 0.01 to 40% by weight of inulin and/or a fructooligosaccharide; and
(c) from 0 to 15% by weight of a fat inhibitor.

Although not wishing to be bound by theory, it is believed that the active ingredients present in the compositions herein described serve to prevent sugar from entering the blood stream through the intestinal wall. This prevents sugar intake from being stored as fat in fatty cells (adipocytes). Firstly, the alpha-amylase inhibitor is believed to reduce the effects of digestive enzymes such as salivary and pancreatic alpha-amylase which convert "slow" sugar molecules (starches) to "fast" sugar molecules. Secondly, the inulin and/or fructooligosaccharide are believed to act on the disaccharidase enzyme which enables "fast" sugars to pass through the intestinal wall thereby slowing down or preventing intestinal absorption of "fast" sugars. Non-digested sugars are eliminated from the body in the natural way.

The alpha-amylase inhibitor for use in the compositions of the invention may be any compound capable of inhibiting the uptake of carbohydrate from the intestinal tract. Typically such compounds will be "starch blockers" which are able to inhibit the absorption of starch, for example by blocking the action of pancreatic alpha-amylase, an enzyme which converts large starch molecules ("slow" sugars) in the intestine to small, "fast" sugar molecules which can be directly assimilated. Consequently, most of the starch is eliminated from the body by excretion.

Preferably, the alpha-amylase inhibitor is a plant protein, e.g. extracted from legumes, wheat, rye, taro root or mangoes. Particularly preferably the alpha-amylase inhibitor is extracted from the kidney bean species Phaseolus Vulgaris, e.g. the red kidney bean. Especially preferred is Phaseolamin™, a glycoprotein extracted from Phaseolus Vulgaris having a molecular weight in the range of 42,000 to 49,000 with approximately 8% of its molecular weight consisting of carbohydrates. Phaseolamin™ comes in two grades which are available from Leuven Bioproducts, a pure L4(PHA-L) form and a mixture of all isoforms (PHA-E: Mix of E4, E3L, E2L2, EL3). Both forms of the extract are suitable for use in the present invention. The alpha-amylase inhibitor will typically be used in an amount of up 35% by weight, preferably 20 to 35% by weight, particularly preferably 25 to 35% by weight, e.g. 30 to 35% by weight.

Compounds capable of reducing absorption of "fast" sugars from the small intestine are inulin and fructooligosaccharides. Preferred compositions according to the invention are those comprising one or more fructooligosaccharides. Mixtures of inulin and fructooligosaccharides may also be used.

Inulin and fructooligosaccharides (FOS) are natural constituents of many common foods, including plants, vegetables, fruits and cereals, e.g. dahlia, helianthus, asparagus, banana, chicory, dandelion, garlic, globe artichoke, Jerusalem artichoke, leek, onion, barley, rye, salsify and wheat. The highest concentrations, on a fresh-weight basis, of inulin and FOS are found within members of the family Compositae (chicory, dandelion, Jerusalem artichoke; also Dahlia). Chicory roots and Jerusalem artichoke are the main sources of inulin and FOS.

Inulin and fructooligosaccharides are not hydrolysed by enzymes in the small intestine and pass through the small intestine unchanged. These are therefore considered to be non-digestible carbohydrates which form part of the dietary fibre component of the diet. These do not increase the insulin level in the blood and their direct calorific value is negligible. Inulin and fructooligosaccharides have the added advantage that they are slightly sweet in flavour and so serve to mimic the sweetening effect of sugar. These therefore rapidly saturate sweet taste receptors on the tongue (thereby quickly reducing the yearning for sugar) while containing few calories. Inulin is also believed to suppress appetite.

Inulin, which consists of molecules which are linear chains of fructose units terminated by a glucose unit, is mainly extracted from endive root. Industrially, inulin is obtained from chicory roots (*Chicorium intybus*) by hot water extraction, followed by refining and spray-drying. Inulin is available under the trade name Raftiline from Orafti SA, Belgium and Fruitafit® from Imperial-Suiker Unie, LLC, Texas.

Fructooligosaccharides comprise linear GFₓ and Fₓ chains (wherein G is a glucosyl moiety, F is a fructosyl moiety and x is the number of fructose units linked together through β(2,1) bonds) with a degree of polymerisation (total number of fructose or glucose units) ranging from 2 to 8, with an average value of about 4. Fₓ oligomers (also termed inulooligosaccharides, inulofructosaccharides or oligofructose) are generally obtained by partial enzymatic or chemical hydrolysis of inulin. The individual compounds are known as inulobiose (F₂), inulotriose (F₃), inulotetraose (F₄), etc. GFₓ oligomers can be obtained by simple aqueous extraction from many plant species, in particular from members of the Gramineae, Liliaceae and Compositae families. They can also be prepared enzymatically from sucrose. For each member of the oligomer series a number of isomers are known. For example, GF₂ can be 1-kestose, 6-kestose and neokestose. Known isomers for GF₃ are nystose, bifurcose and neobifurcose. As used herein, the term "fructooligosaccharide" is intended to encompass any compound or mixture of compounds selected from the group consisting of GFₓ and Fₓ oligomers.

Fructooligosaccharides obtained through partial enzymatic hydrolysis of inulin are available under the trade name Raftilose from Orafti SA, Belgium. These are also available under the trade name Actilight from Beghin-Meiji Industries, France or Imperial-Suiker Unie, LLC. Texas.

The compound capable of reducing absorption of "fast" sugars from the small intestine will generally be used in an amount of up 35% by weight, preferably 20 to 35% by weight, particularly preferably 25 to 35% by weight, e.g. 30 to 35% by weight.

Whilst the invention has been described with particular reference to the use of inulin, it extends also to the use of inulin analogs, such as for example polyfructosan.

In a preferred aspect of the invention the compositions herein described may further comprise at least one fat inhibitor. As used herein, the term "fat inhibitor" is intended to define any physiologically acceptable compound capable of controlling blood lipid levels, for example by reducing or preventing the absorption of dietary fat from the small intestine and/or increasing the metabolism of fat in the body (i.e. able to effect the break-down of fat cells in the adipose tissue). Fat inhibitors suitable for use in the invention may also serve as appetite suppressants, e.g. by increasing the production and storage of glycogen.

In general, the fat inhibitor for use in the invention will be a compound capable of reducing or preventing the uptake of fat from the small intestine. Examples of suitable fat inhibitors include Garcinia cambogia, Garcinia hanburyi (Gamboge) and Garcinia mangostana extracts. Garcinia cambogia is particularly preferred.

Some fibres have the ability to entrap fats in their gelatinous matrix and so prevent their absorption. Chitosan is a fibre which is particularly effective in preventing fat absorption and which may therefore find use in the invention. Chitosan is an amino polysaccharide which has the ability to bind lipids in the stomach before they are absorbed through the digestive system into the blood stream. Chitosan-bound fat leaves the intestinal tract without entering the blood stream. Chitosan's fat entrapment properties can be enhanced by its use in combination with D- or L-ascorbic acid or citric acid.

The fat inhibitor, where present, will generally be used in an amount of up 10% by weight, preferably 5 to 10% by weight.

Generally, the compositions of the invention will comprise at least one pharmaceutically acceptable carrier, diluent or excipient. The active ingredients in such compositions may comprise from 0.1 to about 99% by weight, preferably from 40 to 90% by weight, e.g. from 50 to 80% by weight, of the formulation. By "pharmaceutically acceptable" is meant that the ingredient must be compatible with other ingredients of the compositions as well as physiologically acceptable to the patient.

The compositions herein described may be taken as a dietary supplement for weight control (i.e. to maintain a weight condition) or for losing weight. These may also find use in the treatment of medical conditions associated with weight gain, e.g. in treating obesity. Different formulations will be possible depending on the desired result. Such formulations may be readily determined by those skilled in the art. Typically, the compositions herein described should be used in conjunction with appropriate food reduction and an exercise program.

For dietary control, the composition will typically be taken at least three times a day, preferably immediately before or after meals which contain sugar in any form. Most preferably the composition may be taken up to one hour before or after each meal, most preferably up to 30 minutes before or after each meal. Particularly preferably the composition will be taken before meals. For losing weight, the composition may be taken more frequently. Compositions intended for use in losing weight will generally comprise at least one fat inhibitor, preferably Garcinia cambogia extract.

Viewed from a further aspect the invention thus provides a composition as herein described for use as a medicament, preferably for use in maintaining a weight condition, for losing weight (e.g. in a method of dieting) or for treating a condition associated with weight gain (e.g. obesity).

viewed from a yet further aspect the invention provides the use of a composition as herein described in the manufacture of a medicament for use in a method of maintaining a weight condition, a method of losing weight (e.g. a method of dieting) or a method of treating a condition associated with weight gain (e.g. obesity).

Viewed from a yet still further aspect the invention provides a method of weight loss or a method of preventing weight gain whereby to improve bodily appearance, e.g. a method of dieting, said method comprising administering (e.g. orally) into the gastrointestinal tract of a human or non-human, preferably mammalian, animal body an effective amount of a composition as herein described.

In another aspect the invention provides a method of treating obesity, said method comprising administering (e.g. orally) into the gastrointestinal tract of a human or non-human, preferably mammalian, animal body an effective amount of a composition as herein described.

In some cases it may be beneficial to administer the individual components of the formulation separately. In another aspect the invention thus provides products containing an alpha-amylase inhibitor and separately a physiologically acceptable compound capable of reducing intestinal absorption of "fast" sugars (preferably a compound selected from the group consisting of inulin, fructooligosaccharides and mixtures thereof) for simultaneous, separate or sequential use, for example in a method of maintaining a weight condition, in a method for losing weight (e.g. a method of dieting) or for treating a condition associated with weight gain, e.g. obesity.

The compositions according to the present invention may be formulated in conventional manner using readily available pharmaceutical or veterinary aids. Thus the active ingredients may be incorporated, optionally together with other active substances, with one or more conventional carriers, diluents and/or excipients, to produce conventional galenic preparations such as tablets, pills, powders, capsules, solutions, dispersions, suspensions, emulsions, syrups, etc. However, tablets or capsules will generally be preferred.

Examples of suitable carriers, excipients, and diluents are lactose, dextrose, sucrose, sorbitol, mannitol, starches, gum acacia, calcium phosphate, aglinates, tragacanth, gelatin, calcium silicate, microcrystalline cellulose, polyvinylpyrrolidone, cellulose, water syrup, water, water/ethanol, water/glycol, water/polyethylene, glycol, propylene glycol, methyl cellulose, methylhydroxybenzoates, propyl hydroxybenzoates, talc, magnesium stearate, mineral oil or fatty substances such as hard fat or suitable mixtures thereof. The compositions may additionally include lubricating agents, wetting agents, emulsifying agents, suspending agents, binders, fillers, sweetening agents, viscosity modifiers (e.g. gelling agents, gums and other thickeners), essential nutrients (e.g. vitamins, soluble and insoluble fibre and minerals), colouring agents, pH modifiers (e.g. buffering agents), preservatives, flavours, etc.

The formulations may be formulated so as to provide quick, sustained or delayed release of the active ingredients after administration to the patient by use of procedures well known in the art. For example, when provided in the form of a tablet these may be provided with a gastro-resistant coating, for example comprising a mixture of cellulose acetophtalate and titanium dioxide.

The compositions of the invention may be administered by any suitable method known in the art. However, these are primarily intended for oral ingestion and as such may contain additional sweeteners and flavours to enhance their acceptability to the consumer. Examples of suitable sweeteners include natural sweeteners, e.g. mono-, di- and polysaccharides, for example sucrose, fructose, glucose or sugar alcohols such as sorbitol, mannitol, xylitol, etc., or artificial sweeteners, e.g. aspartame, saccharin and saccharin salts. A single sweetener or two or more sweeteners may be used. The content of sweetener will depend on the content of inulin and/or FOS already present in the compositions and will be readily determined by the person skilled in the art.

The compositions are preferably formulated in a unit dosage form, e.g. with each dosage containing from about 0.1 to about 500mg of the active ingredients.

The precise dosage of the active ingredients and the length of treatment will depend upon a number of factors including, for example, the age and weight of the consumer or patient, the specific condition being treated and its severity (e.g. when treating medical conditions such as obesity). In any event, the correct dose will be readily determined by the person skilled in the art. For example, in a 80 kg subject it is envisaged that two to three 650 mg tablets comprising approximately 450 mgs of active agents may be administered up to three times daily.

Treatment may be continued until the desired weight loss is achieved. Typically, duration of treatment will be at least a month, although this may be prolonged depending on the desired result.

Although the invention has been described primarily with reference to dietary control for humans, the compositions herein described may find use for non-human animals, e.g. canine, feline, bovine, equine, etc. When used for treating animals the quantity of the composition will vary depending on the type of animal.

Preferred embodiments of the invention will now be described by way of the following non-limiting Examples:

### Example 1 - Tablet

A tablet is prepared using the following ingredients:

| | % by weight |
|---|---|
| Phaseolus Vulgaris extract | 30.77 |
| Fructooligosaccharides | 30.77 |
| Microcrystalline Cellulose | 23.08 |
| Garcinia cambodgia extract | 7.70 |
| Tricalcium Phosphate | 4.61 |
| Magnesium stearate | 2.00 |
| Silica | 1.07 |

The components are blended and compressed to form tablets each weighing 650 mg.

### Example 2 - Tablet

A tablet is prepared using the following ingredients:

| | % by weight |
|---|---|
| Phaseolus Vulgaris extract | 30.77 |
| Inulin | 30.77 |
| Microcrystalline Cellulose | 23.08 |
| Tricalcium Phosphate | 4.61 |
| Magnesium stearate | 9.70 |
| Silica | 1.07 |

The components are blended and compressed to form tablets each weighing 650 mg.

## Claims

1. An orally administrable composition comprising at least one alpha-amylase inhibitor together with at least one compound selected from the group consisting of inulin derived from chicory, fructooligosaccharides and mixtures thereof and optionally a fat inhibitor.

2. A composition as claimed in claim 1 comprising:
(a) from 0.01 to 40% by weight of an alpha-amylase inhibitor;
(b) from 0.01 to 40% by weight of inulin and/or a fructooligosaccharide; and
(c) from 0 to 15% by weight of a fat inhibitor.

3. A composition as claimed in any one of claims 1 or 2 wherein said alpha-amylase inhibitor is a plant protein.

4. A composition as claimed in any one of claims 1 or 2 wherein said alpha-amylase inhibitor is a glycoprotein extracted from Phaseolus Vulgaris having a molecular weight in the range of 42,000 to 49,000 with approximately 8% of its molecular weight consisting of carbohydrates.

5. A composition as claimed in any one of claims 1 to 4 wherein said fat inhibitor is selected from the group consisting of Garcinia cambogia, Garcinia hanburyi (Gamboge) and Garcinia mangostana extracts.

6. A composition as claimed in claim 5 wherein said fat inhibitor is Garcinia cambogia.

7. A composition as claimed in any one of claims 1 to 6 comprising 25 to 35% by weight of alpha-amylase inhibitor.

8. A composition as claimed in any one of claims 1 to 7 comprising 25 to 35% by weight of inulin, fructooligosaccharides or mixtures thereof.

9. A composition as claimed in any one of claims 1 to 8 comprising 5 to 10% by weight of fat inhibitor.

10. Use of a composition as claimed in any one of claims 1 to 9 for the manufacture of a medicament for treating obesity, preventing weight gain whereby to improve bodily appearance, or inducing weight loss.

11. Use as claimed in claim 10 wherein said composition is administered orally.

## Patentansprüche

1. Oral verabreichbare Zusammensetzung, umfassend wenigstens einen α-Amylase-Inhibitor zusammen mit wenigstens einer unter aus Zichorie gewonnenem Inulin, Fructooligosacchariden und Gemischen davon ausgewählten Verbindung und gegebenenfalls einen Fettinhibitor.

2. Zusammensetzung nach Anspruch 1, umfassend
(a) 0,01 bis 40 Gew.-% α-Amylase-Inhibitor;
(b) 0,0 bis 40 Gew.-% Inulin und/oder Fructooligosaccharid; und
(c) 0 bis 15 Gew.-% Fettinhibitor.

3. Zusammensetzung nach Anspruch 1 oder 2, wobei es sich bei dem α-Amylase-Inhibitor um ein Pflanzenprotein handelt.

4. Zusammensetzung nach Anspruch 1 oder 2, wobei es sich bei dem α-Amylase-Inhibitor um ein aus *Phaseolus vulgaris* extrahiertes Glykoprotein mit einem Molekulargewicht im Bereich von 42.000 bis 49.000 handelt, wobei etwa 8 % seines Molekulargewichts aus Kohlenhydraten bestehen.

5. Zusammensetzung nach einem der Ansprüche 1 bis 4, wobei der Fettinhibitor unter *Garcinia cambogia, Garcinia hanburyl* (Gummigutt) und- *Garcinia mangostana*-Extrakten ausgewählt ist.

6. Zusammensetzung nach Anspruch 5, wobei es sich bei dem Fettinhibitor um *Garcinia cambogia* handelt.

7. Zusammensetzung nach einem der Ansprüche 1 bis 6, umfassend 25 bis 35 Gew.-% α-Amylase-Inhibitor.

8. Zusammensetzung nach einem der Ansprüche 1 bis 7, umfassend 25 bis 35 Gew.-% Inulin, Fructooligosaccharide oder Gemische davon.

9. Zusammensetzung nach einem der Ansprüche 1 bis 8, umfassend 5 bis 10 Gew.-% Fettinhibitor.

10. Verwendung einer Zusammensetzung nach einem der Ansprüche 1 bis 9 zur Herstellung eines Arzneimittels zur Behandlung von Fettleibigkeit, Verhütung einer Gewichtszunahme zum Zweck der Verbesserung der körperlichen Erscheinung oder zur Herbeiführung eines Gewichtsverlusts.

11. Verwendung nach Anspruch 10, wobei die Zusammensetzung oral verabreicht wird.

## Revendications

1. Une préparation administrable par voie orale et contenant au moins un inhibiteur de l'A-amylase, au moins un constituant du groupe de l'inuline obtenu d'endive, des fructooligosacchrides ou des mélanges à base de ces deux substances et éventuellement un inhibiteur de la graisse.

2. Une préparation conforme au paragraphe 1 et :
(a) dont 0,01 à 40% du poids sont un inhibiteur de l'A-amylase ;
(b) dont 0,01 à 40% du poids sont de l'inuline et/ou un fructooligosaccharide ;
(c) et dont 0 à 15% du poids sont un inhibiteur de la graisse.

3. Une préparation conforme au paragraphe 1 ou 2 et dont l'inhibiteur de l'A-amylase est une protéine d'origine végétale.

4. Une préparation conforme au paragraphe 1 ou 2 et dont l'inhibiteur de l'A-amylase est une glycoprotéine extraite de la Phaseolus Vulgaris dont le poids moléculaire est compris entre 42.000 et 49.000 et contient environ 8% de carbohydrates.

5. Une préparation conforme à l'un des paragraphes 1 à 4 et dont l'inhibiteur de la graisse est un extrait de Garcinia cambodgia, de Garcinia hanburyi (Camboge) ou de Garcinia mangostana.

6. Une préparation conforme au paragraphe 5 et dont l'inhibiteur de la graisse est de la Garcinia cambodgia.

7. Une préparation conforme à l'un des paragraphes 1 à 6 et dont 25 à 35% du poids sont un inhibiteur de l'A-amylase.

8. Une préparation conforme à l'un des paragraphes 1 à 7 et dont 25 à 35% du poids sont de l'inuline, des fructooligosaccharides ou des mélanges à base de ces substances.

9. Une préparation conforme à l'un des paragraphes 1 à 8 et dont 5 à 10% du poids sont un inhibiteur de la graisse.

10. Une préparation conforme à l'un des paragraphes 1 à 9, utlilisée pour la fabrication d'un médicament destiné à :
- traiter l'obésité,
- empêcher une prise de poids en vue l'améliorer l'apparence physique,
- ou faire perdre du poids.

11. Une préparation utilisée conformément au paragraphe 10 et administrée par voie orale.
